**Europäisches Patentamt**

**European Patent Office**

**Office européen des brevets**

(11) Veröffentlichungsnummer: **0 225 617 B1**

(12)

# EUROPÄISCHE PATENTSCHRIFT

(45) Veröffentlichungstag der Patentschrift:
28.06.89

(21) Anmeldenummer: 86116922.5

(22) Anmeldetag: 05.12.86

(51) Int. Cl.⁴: **C07C 21/06**, C07C 17/24

(54) Verbessertes Verfahren zur Herstellung von Vinylchlorid durch thermische Spaltung von 1,2-Dichlorethan.

(30) Priorität: 06.12.85 DE 3543222

(43) Veröffentlichungstag der Anmeldung:
16.06.87 Patentblatt 87/25

(45) Bekanntmachung des Hinweises auf die Patenterteilung:
28.06.89 Patentblatt 89/26

(84) Benannte Vertragsstaaten:
BE DE FR GB IT NL SE

(56) Entgegenhaltungen:
EP-A- 0 180 925
EP-A- 0 180 995
DE-A- 2 913 030
DE-A- 3 112 011
DE-B- 2 349 838

(73) Patentinhaber: WACKER-CHEMIE GMBH,
Prinzregentenstrasse 22, D-8000 München 22(DE)

(72) Erfinder: Schmidhammer, Ludwig, Dr. Dipl.-Chem.,
Pappelweg 5, D-8261 Haiming(DE)
Erfinder: Dummer, Gerhard, Dipl.-Ing. (U),
Lohnerstrasse 12, D-8269 Burgkirchen/Alz(DE)
Erfinder: Haselwarter, Klaus, Dipl.-Ing.(FH),
Hauptstrasse 15b, D-8261 Emmerting(DE)
Erfinder: Klaus, Hermann, Dipl.-Ing.(FH),
Lindenstrasse 4, D-8261 Marktl(DE)
Erfinder: Strasser, Rudolf, Dr. Dipl.-Chem.,
Lindacherstrasse 58, D-8263 Burghausen(DE)

**Beschreibung**

Die Erfindung betrifft ein verbessertes und wirtschaftlicheres Verfahren zur Herstellung von Vinylchlorid (VCM = Vinylchloride monomer) durch sogenanntes Deep Cracking von 1,2-Dichlorethan (EDC = Ethylene Dichloride) bei moderaten Temperaturen am Coilende der Reaktionszone. Unter gleichzeitiger, wirtschaftlicher Ausnutzung des Wärmeinhaltes der Rauchgase der Spaltofenbefeuerung für die Erzeugung von EDC-Flash-Dampf sowie zur Überhitzung der für die Spaltofenbefeuerung benötigten Verbrennungsluft kann man dadurch bei reduziertem Betriebsmittelverbrauch für die EDC-Verdampfung in den Spaltofen und für die Bahandlung des nicht umgesetzten EDC's sowie bei reduziertem spezifischem Heizmittelverbrauch für die Spaltofenbefeuerung die Kapazität eines bestehenden Spaltofens ohne große Investitionen auf etwa 150 % der ursprünglichen Designauslegung erhöhen, ohne daß die Nebenprodukt- und Koksbildung zunimmt bzw. sich die Qualität des erzeugten VCM's verschlechtert. Darüberhinaus wird durch diese Erfindung ein nicht unwesentlicher Beitrag zum allgemeinen Umweltschutz geleistet, weil die Abgastemperatur der Spaltofenbefeuerung dadurch erheblich abgesenkt wird.

Bekanntlich wird VCM großtechnisch durch unvollständige thermische Spaltung von gereinigtem EDC hergestellt. Dabei wird EDC in Dampfform in einem Pyrolyseofen indirekt erhitzt und bei Temperaturen von 400 °C bis 600 °C unter einem Druch von 15 bis 36 bar abs. zu VCM und Chlorwasserstoff gespalten, wobei durch entsprechende Temperaturregulierung im Spaltofen Umsätze von nur 50 bis maximal 60 %, bezogen auf den Gesamtdurchsatz an dampfförmigem EDC, eingehalten werden (vgl. J. Schulze, M. Weiser, Chem. Industrie XXXVI, August 1984, Seite 469). Daher müssen erhebliche Mengen an nicht umgesetztemEDC in die Spaltreaktion zurückgeführt werden, wobei zuvor eine sorgfältige Renigung dieses nicht umgesetzten EDC's durch Abtrennung von gebildetem Vinylacetylen, 1,3-Butadien, Chloropren, 1-Chlorbutadien-1,3 und zahlreichen anderen Chlorkohlenwasserstoffnebenprodukten sowie Aromaten erforderlich ist, weil diese Nebenprodukte teilweise inhibierend wirken und teils auch die Kohlenstoff- und Teerabscheidung begünstigen. Bei Umsetzungsgraden von nur 50 bis maximal 60 %, bezogen auf den Gesamtdurchsatz an EDC, müssen beispielsweise 1,06 bis 1,6 Tonnen (t) nicht umgesetztes EDC pro Tonne erzeugtes VCM zusammen mit der für die jeweilige VCM-Erzeugung benötigten spezifischen EDC-Menge von etwa 1,6 t EDC pro t VCM destillativ gereinigt und in den Spaltofen verdampft werden, d.h. also, es resultiert ein durchschnittlicher spezifischer Energieverbrauch für die Verarbeitung (handling) von insgesamt 2,66 bis 3,2 t EDC pro t VCM-Erzeugung.

Ein Ziel der Erfindung ist es daher, durch Übergang auf deep cracking, d.h. durch Einstellung von EDC-Umsätzen von 60 bis 70 %, bezogen auf den gesamten EDC-Durchsatz, den spezifischen Anfall an nicht umgesetztem EDC/t VCM-Ausbringung drastisch zu senken auf Werte zwischen 0,68 bis 1,05 t EDC/t VCM-Erzeugung und dadurch in Kombination mit anderen Maßnahmen den spezifischen Betriebsmittelverbrauch für die Verfahrensschritte "Reinigung von nicht umgesetztem EDC" und "Verdampfen von gereinigtem EDC in den Spaltofen" deutlich zu reduzieren, ohne Qualitätseinbußen des erzeugten VCM's bzw. Selektivitätseinbußen des Gesamtprozesses und verkürzte Ofenstandzeiten (Kapazitätseinbuße) bei stark erhöhter VCM-Ausbringung hinnehmen zu müssen.

Gegenstand der Erfindung ist ein Verfahren zur Herstellung von Vinylchlorid durch thermische Spaltung von 1,2-Dichlorethan, das bei der destillativen Reinigung mit einer Temperatur von 125 °C bis 155 °C und entsprechenden Drücken in flüssiger Form anfällt und ohne Zwischenlagerung sowie nach Zusatz von Tetrachlorkohlenstoff als Spaltpromotor in einem außenliegenden Verdampfer verdampft wird, bei Temperaturen von 400 °C bis 600 °C unter einem Druck von 10 bis 16 bar abs. sowie bei Umsätzen von 50 bis 60 % und Verweilzeiten von 0,1 bis 30 Sekunden in der Reaktionszone unter teilweiser Ausnützung des Wärmeinhalts der Rauchgase der Spaltofenbefeuerung zur Vorwärmung von flüssigem 1,2-Dichlorethan auf nahezu Siedetemperatur entsprechenden Druckes vor Eintritt in den externen Verdampfer und unter Verwendung von auf etwa 100 °C vorgewärmter Luft für die Spaltofenbefeuerung. Das Verfahren ist dadurch gekennzeichnet, daß man

a) den Gehalt an Tetrachlorkohlenstoff in flüssigem 1,2-Dichlorethan bei Werten zwischen 0,10 und 0,15 Gew.-%, bevorzugt zwischen 0,11 und 0,13 Gew.-%, jeweils bezogen auf 1,2-Dichlorethan, hält und gleichzeitig den Gehalt an Chloroform im flüssigen 1,2-Dichlorethan bei Werten < 200 Gew.-ppm., bezogen auf 1,2-Dichlorethan, einstellt;

b) das 125 bis 155 grädige flüssige und gemäß a) mit Tetrachlorkohlenstoff beladene 1,2-Dichlorethan in der Konvektionszone des Spaltofens unter Ausnützung eines Großteils des Wärmeinhaltes der Rauchgase der Spaltofenbefeuerung bei einem Flüssigkeitsdruck von 15 bis 31 bar abs. auf nahezu Siedetemperatur vorwärmt und nach Austritt aus der Konvektionszone des Spaltofens das vorgewärmte, flüssige 1,2-Dichlorethan auf einen Druck von 10 bis 16 bar abs. entspannt, wobei etwa 18 bis 70 Gew.-% des gesamten 1,2-Dichlorethans verdampfen;

c) das gemäß b) gewonnene dampfförmige 1,2-Dichlorethan vom flüssigen Anteil abtrennt, diesen flüssigen Anteil dann in einem außenliegenden Verdampfer bei einem Druck von 10 bis 16 bar abs. verdampft und die vereinigten Ströme an dampfförmigem 1,2-Dichlorethan, in denen auch der Tetrachlorkohlenstoff in Dampfform zugegen ist, in solchen Mengen in die Reaktionszone des Spaltofens einführt, daß die stündliche Beaufschlagung/m² Spaltrohr querschnitt 1100 bis 1500 t 1,2-Dichlorethan, bevorzugt 1200 bis 1300 t 1,2-Dichlorethan beträgt;

d) 1,2-Dichlorethanumsätze von 60 bis 70 %, bevorzugt 62 bis 66 %, bei einer mittleren Verweilzeit von 10 bis maximal 15 Sekunden, bezogen auf den befeuerten Teil der Reaktionszone, einstellt, indem man den Spaltofen so befeuert, daß im Bereich des Überganges von der Schock- in die Strahlungszone des Reaktionsraumes bereits ein Temperaturniveau von 425 bis 455 °C, bevorzugt 430 bis 440 °C, herrscht, etwa in der Mitte der Reaktionszone Temperaturen von 460 bis 480 °C, bevorzugt 465 bis 470 °C, herrschen und schon bei etwa 75 bis 85 % der gesamten Rohrlänge der Reaktionszone, bestehend aus Schock- und Strahlungsteil, der Rest der gesamten benötigten Energie zugeführt wird, um am Coilende Austrittstemperaturen von 485 bis 510 °C, bevorzugt 495 bis 505 °C, einzuhalten.

Gemäß einer besonderen bevorzugten Ausführungsform des Verfahrens nützt man den Wärmeinhalt der Rauchgase der Spaltofenbefeuerung, welche die Konvektionszone des Spaltofens mit einer Temperatur von 240 bis 540 °C verlassen, zur Überhitzung der etwa 100-grädigen Verbrennungsluft für die Spaltofenbefeuerung auf Temperaturen von 200 bis 500 °C unter gleichzeitigem Abkühlen der Rauchgase auf Temperaturen von 140 bis 180 °C.

Die zu reinigenden bzw. zu verdampfenden Mengen an EDC betragen erfindungsgemäß in summa nur noch 2,28 bis 2,65 t EDC/t VCM-Erzeugung, reduzieren sich dadurch also um rund 14 bis 28 %, bezogen auf eine konventionelle Fahrweise mit EDC-Umsätzen von 50 bis maximal 60 %. In Kombination mit der erfindungsgemäßen Erzeugung von EDC-Flash-Dampf ergibt sich somit eine mittlere Einsparung von ca. 0,35 t des im allgemeinen zur Beheizung solcher Verfahren verwendeten Dampfs pro t VCM-Erzeugung.

Für die Befeuerung eines Spaltofens sind - klammert man den Energiebedarf für die EDC-Verdampfung aus - je t des zu er zeugenden VCM etwa 3 Giga-Joule (GJ) an Wärmeenergie aufzubringen, wovon rund 80 % dieser Wärmemenge für die Vorwärmung von flüssigem EDC in der Konvektionszone des Spaltofens auf nahezu Siedetemperatur bei einem Druck von 10 bis 16 bar abs., für die Überhitzung von dampfförmigem EDC auf Spalttemperatur sowie für den endothermen Spaltprozeß benötigt werden. Rund 20 % davon gehen jedoch mit den Rauchgasen der Spaltofenbefeuerung verloren, da eine Wärmerückgewinnung wegen des dabei sich einstellenden, relativ niedrigen Rauchgastemperaturniveaus von etwa 270 °C bis 330 °C kaum wirtschaftlich ist.

Es sind daher, einer wirtschaftlichen Notwendigkeit in der heutigen Zeit folgend, in jüngster Zeit Verfahren vorgeschlagen worden, einen Teil der für die Spaltung von EDC erforderlichen Wärmemenge - soweit sie als Wärmeinhalt der Spaltgase auftritt - zurückzugewinnen. Daneben wird in einigen Fällen auch die Rückgewinnung von Abwärme aus den Rauchgasen einer Spaltofenbefeuerung unter Erzeugung von Wasserdampf praktiziert. Wegen der relativ niedrigen Rauchgastemperatur sind derartige Verfahren jedoch wenig wirtschaftlich. Wenn man auch gemäß der Deutschen Patentanmeldung P 34 40 685.9 (= EP-A 0 180 925) die Rauchgastemperatur anhebt, sind für eine Dampferzeugung immer noch relativ hohe Investitionskosten erforderlich.

Ein weiteres Ziel der Erfindung ist est daher, die Rauchgastemperatur im Oberteil der Feuerbox vor Eintritt in die Konvektionszone des Spaltofens durch gezieltes Einbringen von Strahlungsenergie an bestimmten Stellen der Reaktionszone so anzuheben, daß das in der Konvektionszone vorzuwärmende flüssige EDC weit über die einem Druck von 10 bis 16 bar abs. entsprechende Siedetemperatur bei entsprechenden Flüssigkeitsdrücken vorgewärmt wird, um durch Entspannen auf einen Druck von 10 bis 16 bar abs. EDC-Flash-Dampf zu erzeugen und durch Kombination mit den in der Deutschen Patentanmeldung P 34 40 685.9 beschriebenen Maßnahmen das Rauchgastemperaturniveau nach Austritt aus der Konvektionszone des Spaltofens so hoch zu halten, daß die Verbrennungsluft für die Spaltofenbefeuerung, die nach bereits bekannten Methoden auf etwa 100 °C vorgewärmt ist, durch Wärmetausch mit den Rauchgasen überhitzt werden kann, um kostbare und nicht in unbegrenzten Mengen vorhandene Primärenergie, z.B. in Form von Erdgas, einzusparen.

Bekanntlich ist der Umsatz bei der thermischen Spaltung von EDC weitgehend von der Temperatur abhängig (vgl. AT-B-294026). Aus wirtschaftlichen Gesichtspunkten wird generell ein möglichst hoher EDC-Umsatz angestrebt. Erhöhter Umsatz hat jedoch zur Folge, daß auch die Spaltnebenprodukte und die Koksbildung zunehmen. Als Nebenprodukte können gesättigte und ungesättigte aliphatische Kohlenwasserstoffe sowie aromatische Kohlenwasserstoffe, wie z.B. Vinylacetylen, Butadien-1,3, 2-Chlorbutadien-1,3, 1-Chlorbutadien-1,3, isomere Dichlorbutadiene, Tetrachlorbutadiene, 1,1-Dichlorethylen, 1,1-Dichlorethan, Methylchlorid, Chloroform, Ethylchlorid, Tetrachlorkohlenstoff und Benzol nachgewiesen werden. Da sie Siedepunkte dieser Verunreinigungen und von EDC teilweise sehr nahe beieinander liegen, tritt eine allmähliche Anreicherung der genannten Nebenprodukte in dem im Kreis geführten nicht umgesetzten EDC ein. Die zunehmende unkontrollierte Anreicherung der Stoffe hat mehrere negative Auswirkungen. Erstens verkokt die Spaltschlange (Coil) schneller, so daß im Zusammenspiel mit erhöhten Reaktionstemperaturen, die für eine Umsatzsteigerung erforderlich sind, vermehrt Koks entsteht. In der Spaltschlange vermehrt abgelagerter Koks verursacht einen höheren Druckabfall über das Reaktionssystem. Somit wird eine häufigere Entkokung der Spaltschlange erforderlich, was einer Kapazitätsminderung der Anlage gleichkommt. Daneben bewirken sowohl erhöhte Reaktionstemperaturen, die für eine Umsatzsteigerung erforderlich sind, als auch vermehrte Koksablagerungen in der Spaltschlange automatisch erhöhte Wandtemperaturen des Spaltrohrmaterials, weil zum einen mit der Anhebung der Reaktionstemperatur aufgrund des endothermen Spaltprozesses die Wandtemperatur entsprechend erhöht werden muß, um eine genügend hohe Temperaturdifferenz für den benötigten spezifischen Wärmefluß einzustellen und weil zum anderen aufgrund der Wärmeisolationseigenschaften von Koks bei stärkerer Koksablagerung die benötigte Temperaturdifferenz zwischen Produktstrom und Rohrwand noch entsprechend höher sein muß. Erhöhte Wandtemperaturen verursachen jedoch Materialschäden, wie Hochtemperaturkorrosion durch HCl, Aufkokung durch Ausscheiden von Carbiden und im schlimmsten Fall "Metal dusting", wodurch die Spaltrohre aufbrechen können und es zu Bränden und Explosionen kommen kann. Daneben erhöhen sich bei Zunahme der Spaltnebenprodukte generell auch die Verunreinigungen im VCM. Eine destillative Abtrennung von Butadien-1,3, Vinylacetylen und Methylchlorid aus dem VCM ist jedoch nur mit einer sehr aufwendigen Destillationsanordnung zu erreichen.

Aus der US-A-2 755 315 ist bekannt, daß die Spaltreaktion durch Zusatz von Tetrachlorkohlenstoff als Spaltinitiator beschleunigt werden kann. Dies äußert sich entweder dadurch, daß bei vergleichbarer Reaktionstemperatur der EDC-Umsatz erhöht oder daß bei vergleichbarem EDC-Umsatz die Reaktionstemperatur abgesenkt werden kann. Als untere Grenze für die katalytische Wirksamkeit von Tetrachlorkohlenstoff wird in obengenannter US-Patentschrift 0,5 %, bezogen auf EDC-Einsatz, angegeben. Die Anwesenheit so großer Mengen an Tetrachlorkohlenstoff bei der Spaltreaktion begünstigt jedoch auch die Bildung unerwünschter Nebenprodukte, wie z.B. Chloroform und Methylchlorid. Methylchlorid siedelt sich aufgrund seines Siedepunktes überwiegend im VCM an und ist nur mit großem technischem Aufwand vom VCM abzutrennen. Chloroform gelangt über das nicht umgesetzte, im Kreis geführte EDC trotz vorheriger destillativer Reinigung wieder in den Spaltprozeß zurück, weil es im Verein mit anderen, im rohen EDC vorhandenen leichtsiedenden Komponenten nur unter erschwerten Bedingungen destillativ abtrennbar ist, und verursacht dort eine extrem starke Koksbildung mit all ihren schädlichen Begleiterscheinungen, wobei ein besonders grobkörniger strukturierter Koks entsteht, der teilweise sehr schlechte Hafteigenschaften hat und somit mit dem Produktstrom aus dem Spaltrohr ausgeblasen wird und so in der nachfolgenden Produktquenche zu vermehrten Koksablagerungen und Erosionen führt.

Nach der US-A-3 222 407 werden als Reaktionsbeschleuniger 0,05 bis 5 % Chlor zusammen mit 100 bis 2500 ppm Tetrachlorkohlenstoff verwendet. Bei den hohen Temperaturen der Spaltreaktion gibt Chlor jedoch Anlaß zu starker Korrosion des Spaltrohrmaterials.

Gemäß DE-B 23 49 838 wird die thermische Spaltung von EDC in Gegenwart von 0,01 bis 0,3 Gew.-% Tetrachlorkohlenstoff, bezogen auf EDC, durchgeführt. Wir haben jedoch festgestellt, daß bei Konzentrationen < 0,1 Gew.-% an Tetrachlorkohlenstoff in EDC der beschleunigende Effekt von Tetrachlorkohlenstoff sehr gering ist, vor allem bei relativ kurzen mittleren Verweilzeiten im beheizten Teil der Reaktionszone, was in Kombination mit der Änderung der Ofenbeheizung ein Gegenstand dieser Erfindung ist. Ferner nimmt schon bei Konzentrationen > 0,15 Gew.-% an Tetrachlorkohlenstoff im EDC die Koksbildung so dramatisch zu, daß innerhalb von nur wenigen Wochen ein deutlicher Anstieg im Druckabfall über die Reaktionszone erkennbar ist, vor allem wenn durch die erfindungsgemäße Anordnung der Spaltofenbefeuerung die Spaltreaktion schon relativ früh einsetzt.

In Kombination mit der erfindungsgemäßen Anordnung der Spaltofenbefeuerung bzw. mit verkürzten mittleren Verweilzeiten im befeuerten Teil der Reaktionszone wirkt sich erfindungsgemäß eine Konzentration von 0,1 bis 0,15 Gew.-%, bevorzugt 0,11 bis 0,13 Gew.-%, Tetrachlorkohlenstoff im EDC als besonders günstig hinsichtlich Erhöhung des EDC-Umsatzes bzw. Absenkung der Reaktionstemperatur am Coilende aus, ohne daß vermehrt unerwünschte Nebenprodukte und Koks anfallen. Vielmehr wird durch die Zugabe von Tetrachlorkohlenstoff im erfindungsgemäßen Bereich in Kombination mit der geänderten Spaltofenbefeuerung überraschenderweise eine Verminderung der Ausbildung von Butadien-1,3, Vinylacetylen und Methylchlorid erreicht. Zur Aufrechterhaltung der erfindungsgemäßen Tetrachlorkohlenstoffkonzentration im EDC bei gleichzeitiger Einhaltung eines Chloroformpegels von < 200 Gew.-ppm. (mg/kg) im EDC gibt es mehrere Möglichkeiten: einmal kann man den Gehalt an Chloroform, das hauptsächlich bei der Ethylenoxichlorierungsreaktion gebildet wird, in gewaschenem, rohem EDC dadurch stark absenken, daß man das sogenannte sekundäre Chloroform, das sich durch kaustische Spaltung von Chloralhydrat, gelöst im Abwasser der Oxichlorierung, während des alkalischen Strippens dieses Abwassers bildet und sich im organischen Stripperkondensat befindet, nach Phasentrennung vom Prozeß abtrennt und einer anderen Verwendung zuführt. Dadurch gelingt es, bei insgesamt reduziertem Chloroformgehalt im Zulauf zur Leichtsiederkolonne bei der Leichtsieder- und Wasserabtrennung aus gewaschenem, rohem EDC, z.B. gemäß DE-B-24 45 371, den Destillationsprozeß so zu steuern, daß im Sumpf dieser Leichtsieder- bzw. Azeotropkolonne und somit auch im gereinigten EDC, das am Kopf der Schwersiederkolonne anfällt, weniger als 200 ppm Chloroform und 1000 bis 1500 ppm (mg/kg) Tetrachlorkohlenstoff anwesend sind. Ferner kann man den gewünschten EDC-Verschnitt erreichen, indem man die Leichtsiederkolonne gemäß DE-A 31 12 011 betreibt. Verfährt man gemäß der Deutschen Patentanmeldung P 34 41 045.7 (= EP-A 0 180 998), so wird die überwiegend Menge des als Spaltpromotor eingesetzten Tetrachlorkohlenstoffs zusammen mit dem nicht umgesetzten EDC wieder in den Spaltofen zurückgeführt, da die Siedepunkte von Tetrachlorkohlenstoff und EDC nahezu identisch sind. Es sind also nur minimale Mengen an Tetrachlorkohlenstoff zu ergänzen, was z.B. durch entsprechende Manipulation der Teilkondensationstemperatur im destillativen Verfahren gemäß DE-B-24 45 371 leicht zu bewerkstelligen ist und am besten über einen on-line-Prozeßchromatographen im Feed-EDC zum Spaltofen überwacht werden kann. Darüberhinaus ist es natürlich auch möglich, Tetrachlorkohlenstoff in entsprechenden Mengen von außen dem EDC zuzusetzen. Dies erfolgt am besten vor dem Vorwärmen und Verdampfen von EDC. Man kann es aber prinzipiell auch nach dem Vorwärmen zusetzen oder direkt in den Spaltreaktor einspeisen.

In Kombination mit der beschleunigten Wirkung von Tetrachlorkohlenstoff im erfindungsgemäßen Zugabebereich erreicht man entsprechend einer weiteren erfindungsgemäßen Maßnahme erhöhte EDC-Umsätze von 60 bis 70 %, bezogen auf EDC-Einsatz, ohne merkliche Anhebung der Austrittstemperatur am Coilende der Spaltzone, wenn man einen Großteil der insgesamt benötigten Wärmeenergie schon relativ früh in die Reaktionszone einbringt und die Befeuerung der Reaktionszone schon bei etwa 75 bis 85 % der gesamten Rohrlänge der Reaktionszone, bestehend aus Schock- und Strahlungszone, abschließt. Dabei war es überraschend, daß trotz des dadurch erfolgenden früheren Einsetzens der Spaltreaktion, d.h. insgesamt verlängerter Verweilzeit von gebildetem VCM im Reaktionssystem, keine erhöhte Nebenprodukt- und Koksbildung beobachtet wird, zumal es allgemein bekannt ist, daß die Weiterreaktion von gebildetem VCM zu störenden Nebenprodukten und Koks sehr stark vom Partialdruck abhängig ist, der mit steigendem EDC-Umsatz jedoch entsprechend zunimmt. Ferner war es überraschend,daß die bei insgesamt verkürzt befeuerter Reaktionszone daraus resultierende, ebenfalls verkürzte mittlere Verweilzeit in der Reaktionszone noch ausreicht, um derart hohe EDC-Umsätze bei moderaten Reaktionstemperaturen am Coilende zu erzielen.

Durch die erfindungsgemäße Änderung der Spaltofenbefeuerung erhöht sich naturgemäß bei sonst gleichbleibendem spezifischem Heizmittelbedarf die Temperatur der aus der Schock- und Strahlungszone aufsteigenden Rauchgase von ca. 600 °C bei konventioneller Spaltofenbefeuerung auf etwa 670 bis 700 °C. Dieses erhöhte Rauchgastemperaturniveau kann daher zur Erzeugung von EDC-Flashdampf verwendet werden, indem man 125 bis 155-grädiges flüssiges EDC, welches z.B. gemäß der Deutschen Patentanmeldung P 34 40 685.9 gewonnen wird, in der Konvektionszone des Spaltofens durch Wärmetausch mit den Rauchgasen der Spaltofenbefeuerung in flüssiger Form entsprechenden Druckes auf Temperaturen bringt, die weit über dem Siedepunkt entsprechend einem Druck von 10 bis 16 bar abs. liegen und hinterher auf 10 bis 16 bar abs. entspannt. Diese Art der Gewinnung von EDC-Flashdampf ist zwar an sich bekannt, ist jedoch nur wirtschaftlich durchzuführen, wenn das flüssige EDC genügend hoch vorgewärmt werden kann, was durch die bereits beschriebene Kombination der erfindungsgemäßen Maßnahmen ohne Erhöhung des spezifischen Heizmittelbedarfs für den Spaltreaktor erreicht werden kann. Man spart dadurch nicht unbeträchtliche Mengen an Heizdampf für die externe EDC-Verdampfung ein bzw. kann dadurch die gewollte Leistungssteigerung einer bestehenden Anlage auch mit einem Engpaß in der EDC-Verdampfung problemlos erreichen. Durch die höhere Vorwärmung des flüssigen EDC's in der Konvektionszone eines Spaltofens würde dem Rauchgas natürlich mehr Wärme entzogen, wenn das flüssige EDC nur mit einer Temperatur von etwa 100 °C, die erfahrungsgemäß notwendig ist, um Taupunktskorrosion am Ende der Rippenrohre der Konvektionszone zu verhindern, einträte. Dadurch wäre eine weitere Ausnützung des Wärmeinhaltes der die Konvektionszone verlassenden Rauchgase aufgrund eines zu niedrigen Temperaturniveaus jedoch in keiner Weise mehr wirtschaftlich. Wenn man das flüssige EDC jedoch mit einer Temperatur von 125 bis 155 °C einbringt, wie dies bei Verwendung des in der Deutschen Patentanmeldung P 34 40 685.9 beschriebenen Destillationsprozesses möglich ist, ist das Temperaturniveau der die Konvektionszone verlassenden Rauchgase trotz erhöhte Flüssig-EDC-Vorwärmung noch so hoch, daß der Wärmeinhalt dieser Rauchgase zur Erzeugung von Dampf bzw. - weil insgesamt wirtschaftlicher, da niedrigere Investitionskosten verursachend - erfindungsgemäß zur Überhitzung etwa 100-grädiger Verbrennungsluft für die Spaltofenbefeuerung verwendet werden kann. Man spart dadurch wertvolle und nicht in unbegrenzten Mengen vorhandene Primärenergie, z.B. in Form von Erdgas, ein.

Die nachfolgend aufgeführten Beispiele sollen den Umfang der Erfindung weiter erläutern. Nm³ bedeutet m³ , auf Normalbedingungen berechnet (0 °C,1013 hPa).

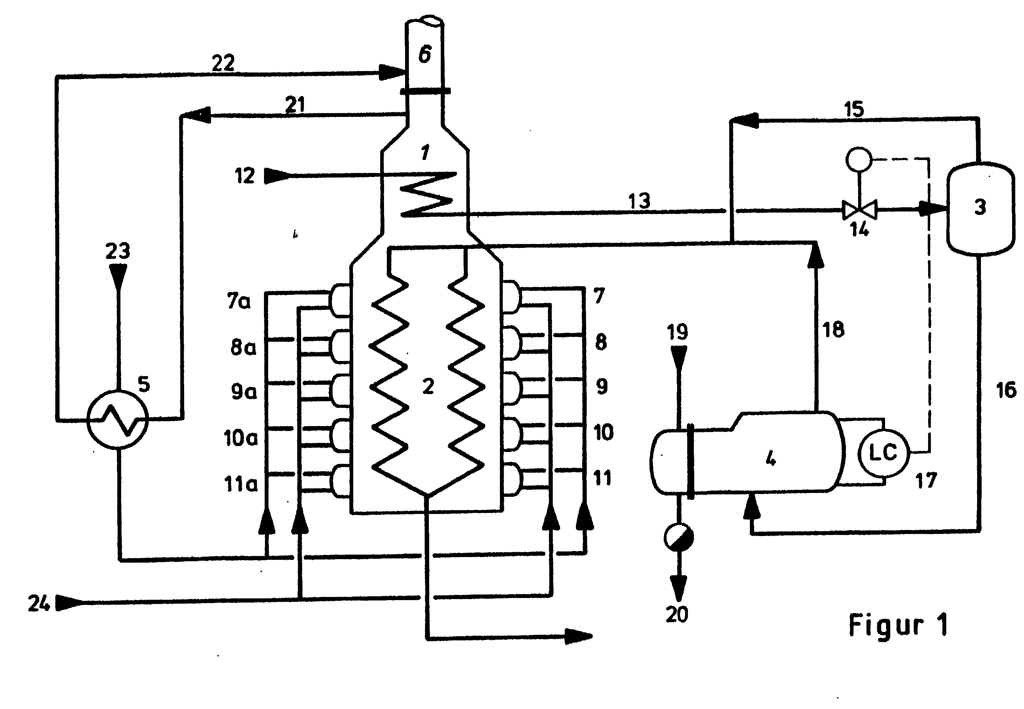

Beispiel 1 (vgl. beiliegende Figur 1)

71 t/h EDC, das unter anderem mit 1220 Gew.-ppm. Tetrachlorkohlenstoff und 130 Gew.-ppm. Chloroform versetzt war und in flüssiger Form bei dem gemäß der Deutschen Patentanmeldung P 34 40 685.9 beschriebenen Destillationsprozeß mit einer Temperatur von etwa 140 °C anfiel, wurden über Leitung 12 in die Konvektionszone 1 des Spaltofens gepumpt und durch Wärmetausch mit den Rauchgasen, die mit einer Temperatur von 672 °C aus der Schock- und Strahlungszone 2 des Spaltofens austraten, bei einem Flüssigkeitsdruck von 26 bar abs. auf 243 °C vorgewärmt, wobei das EDC sich gerade noch in flüssigem Zustand befand. Über Leitung 13 wurde das überhitzte Flüssig-EDC im Strandregelventil 14, das vom Standregler 17 des externen EDC-Verdampfers 4 gesteuert wurde, auf 14 bar abs. entspannt, wobei 28.027 kg/h EDC in den Dampfzustand übergingen. Nach Trennung dieses EDC-Dampfes von 42.973 kg/h noch flüssig gebliebenem EDC im Gasflüssigkeitsabscheider 3 gelangte das restliche Flüssig-EDC standgeregelt über Leitung 16 in den externen Verdampfer 4, der über Leitung 19 mit 5,5 t/h 22 bar Dampf beaufschlagt war (Kondensatabführung über Leitung 20), wo es verdampfte und über Leitung 18 zusammen mit dem EDC-Flashdampf, der über Leitung 15 aus dem Gasflüssigkeitsabscheider 3 austrat, in die Schock- und Strahlungszone 2 des Spaltofens eingeleitet wurde. Der Spaltofen war als Zweipass-System angeordnet. Der innere Rohrdurchmesser der beiden Spaltschlangen betrug 192 mm, woraus eine spezifische Beaufschlagung von 1225 t EDC/m² resultierte. In der Schock-und Strahlungszone 2 des Spaltofens wurden die Spaltrohre über die vier Brennerebenen 7/7a bis 10/10a so befeuert, daß im Bereich des Übergangs von der Schock- in die Strahlungszone bei etwa 30 % der gesamten Rohrlänge der Reaktionszone eine Produkttemperatur von 435 °C herrschte , etwa in der Mitte der Reaktionszone die Produkttemperatur beider Pässe 468 °C betrug und bei etwa 80 % der gesamten Rohrlänge die restliche benötigte Gesamtenergie zugeführt war (d.h. die Brennerebenen 11/11a waren abgeschaltet), wodurch sich am Coilende jedes Passes eine Produktaustrittstemperatur von 497 °C einstellte und die mittlere Verweilzeit im befeuerten Reaktionsraum 11 Sekunden betrug. Die Spaltung erreichte einen Umsatz von 64 %, bezogen auf eingesetztes EDC, entsprechend einer VCM-Ausbringung von 28,5 t/h nach Abtrennung von 17 t/h HCl und 25,5 t/h nicht umgesetztem EDC, wobei folgende VCM-Produktqualität erzielt wurde:
7,0 Gew.-ppm Butadien-1,3
6,8 Gew.-ppm Vinylacetylen
32 Gew.-ppm Methylchlorid
Rest VCM.

Die Rauchgase traten aus der Konvektionszone 1 des Spaltofens mit einer Temperatur von 360 °C aus und wurden über Leitung 21 dem Wärmetauscher 5 zugeführt, in dem 100-grädige Luft bei 23 in einer Menge von 34.500 kg/h - auf 100 °C vorgewärmt, z.B. gemäß der DE-Patentanmeldung P 34 41 080.5 (≙EP-A 0 180 995) - durch Wärmetausch mit den Rauchgasen auf 330 °C überhitzt wurde, bevor sie mit 2.066 Nm³ /h Erdgas, das über Leitung 24 zugeführt wurde, vermischt in den Brennern der Ebenen 7/7a bis 10/10a zur Spaltofenbefeuerung verwendet wurde. Die Rauchgase kühlten sich dabei auf 160 °C ab und wurden über Leitung 22 in den Kamin 6 abgelassen.

Die Standzeit des Spaltofens betrug 7 Monate, d.h. erst nach 7 Monaten Laufzeit mußte wegen koksbedingten Anstiegs des Druckabfalls über die Spaltschlangen der Ofen entkokt werden. Der spezifische Dampfverbrauch für die EDC-Verdampfung betrug 0,19 t/t VCM, der spezifische Erdgasverbrauch 72,5 Nm³ / t VCM. Vergleichsweise betrug in einer konventionell befeuerten Spaltanlage und bei 55 % EDC-Umsatz der spezifische Dampfverbrauch für die EDC-Verdampfung etwa 0,39 t/t VCM und der spezifische Erdgasverbrauch rund 84 Nm³ / t VCM.

## Vergleichsbeispiel A

Es wurde analog Beispiel 1 verfahren, jedoch mit der Ausnahme, daß der Gehalt an Tetrachlorkohlenstoff im EDC 2.000 Gew.-ppm betrug. Bei sonst gleichen Bedingungen erhielt man bei einer geringfügig niedrigeren Produktaustrittstemperatur am Coilende von 495 °C ein VCM, welches folgende Verunreinigungen aufwies:
10 Gew.-ppm Butadien-1,3
9,5 Gew.-ppm Vinylacetylen
40 Gew.-ppm Methylchlorid
Schon nach 6 Wochen stieg die Temperaturdifferenz im Spaltofen durch vermehrte Koksablagerungen so stark an, daß der Durchsatz um 20 % abgesenkt werden mußte, um im Verdampfer 4 noch genügend EDC mit dem zur Verfügung stehenden Heizdampf mit einem Druck von 22 bar verdampfen zu können. Nach 3 monaten Laufzeit mußte der Ofen entkokt werden, da die Leistung immer weiter abfiel.

## Vergleichsbeispiel B

Analog Beispiel 1 wurde EDC mit einem Gehalt von 800 Gew.-ppm Tetrachlorkohlenstoff gespalten. Zur Aufrechterhaltung eines vergleichbaren EDC-Umsatzes von 64 % war die Befeuerung so zu ändern, daß die Produkttemperatur am Coilende 515 °C betrug. Das erzeugte VCM hatte folgende Produktqualität:
15 Gew.-ppm Butadien-1,3
14,5 Gew.-ppm Vinylacetylen
55 Gew.-ppm Methylchlorid
Infolge verstärkter Koksablagerungen aufgrund der erhöhten Austrittstemperatur mußte der Spaltofen nach 3,5 Monaten Laufzeit entkokt werden.

## Vergleichsbeispiel C

Analog Beispiel 1 wurde EDC mit einem Gehalt von 1220 Gew.-ppm Tetrachlorkohlenstoff gespalten, wobei der Chloroformgehalt jedoch 500 Gew.-ppm betrug. Bei sonst gleichen Bedingungen und annähernd gleicher VCM-Qualität entstand vermehrt Koks, der sich teilweise in den Spaltschlangen ablagerte, so daß die Ofenstandzeit nur 5 Monate betrug, zum Teil aber auch in die Quenche ausgetragen wurde und dort Erosionen verursachte, die nach 2 Laufperioden eine Reparatur der erodierten Teile der Quenche durch Auftragsschweißen erforderlich machten.

## Vergleichsbeispiel D

EDC mit einem Tetrachlorkohlenstoff- und Chloroformgehalt analog Beispiel 1 wurde unter konventioneller Spaltofenbefeuerung gespalten, wobei sich der gesamte Erdgasbedarf auf 5 Brennerebenen (also auch zusätzlich auf die Brennerebenen 11/11a) verteilt und sich somit eine mittlere Verweildauer in der befeuerten Reaktionszone von etwa 18 Sekunden ergab. Dabei stellten sich schon bei 58 % EDC-Umsatz und einer spezifischen Beaufschlagung von 1.000 t EDC/m² Rohrquerschnitt folgende Produkttemperaturen bzw. Rauchgastemperaturen ein:
beim Übergang von der Schock- in die Strahlungszone: 390 °C in der Mitte der Reaktionszone: 445°C
am Coilende: ca. 525 °C
Rauchgas am Austritt der Schockzone: 605 °C
Selbst bei Eintritt von 140-grädigem EDC in die Konvektionszone der Spaltzone konnten durch Entspannen von 14 bar auf 10 bar nur 15 % EDC Flashdampf gewonnen werden, wenn auch noch die benötigte Verbrennungsluft von 100 °C auf 330 °C überhitzt werden sollte.

Daraus resultierte ein spezifischer Dampfverbrauch für die EDC-Verdampfung von 0,31 t/t VCM, ein spezifischer Erdgasverbrauch von 74 Nm³ /t VCM und eine Produktausbringung von nur 21 t/h VCM folgender Qualität:

19 Gew.-ppm Butadien-1,3
18 Gew.-ppm Vinylacetylen
65 Gew.-ppm Methylchlorid

Infolge starker Koksablagerungen in den Spaltrohren betrug die Ofenstandzeit nur 2,5 Monate.

## Patentansprüche

1. Verfahren zur Herstellung von Vinylchlorid durch thermische Spaltung von 1,2-Dichlorethan, das bei der destillativen Reinigung mit einer Temperatur von 125 °C bis 155 °C und entsprechenden Drücken in flüssiger Form anfällt und ohne Zwischenlagerung sowie nach Zusatz von Tetrachlorkohlenstoff als Spaltpromotor in einem außenliegenden Verdampfer verdampft wird, bei Temperaturen von 400 bis 600 °C unter einem Druck von 10 bis 16 bar abs. sowie bei Umsätzen von 50 bis 60 % und Verweilzeiten von 0,1 bis 30 Sekunden in der Reaktionszone, unter teilweiser Ausnützung des Wärmeinhalts der Rauchgase der Spaltofenbefeuerung zur Vorwärmung von flüssigem 1,2-Dichlorethan auf nahezu Siedetemperatur entsprechenden Druckes vor Eintritt in den externen Verdampfer und unter Verwendung von auf etwa 100 °C vorgewärmter Luft für die Spaltofenbefeuerung, **dadurch gekennzeichnet,** daß man

a) den Gehalt an Tetrachlorkohlenstoff in flüssigem 1,2-Dichlorethan bei Werten zwischen 0,10 und 0,15 Gew.-%, bezogen auf 1,2-Dichlorethan, hält und gleichzeitig den Gehalt an Chloroform im flüssigen 1,2-Dichlorethan bei Werten unter 200 mg/kg 1,2-Dichlorethan einstellt;

b) das 125 bis 155-grädige flüssige und gemäß a) mit Tetrachlorkohlenstoff beladene 1,2-Dichlorethan in der Konvektionszone des Spaltofens unter Ausnützung eines Großteils des Wärmeinhalts der Rauchgase der Spaltofenbefeuerung bei einem Flüssigkeitsdruck von 15 bis 31 bar abs. auf nahezu Siedetemperatur vorwärmt und nach Austritt aus der Konvektionszone des Spaltofens das vorgewärmte, flüssige 1,2-Dichlorethan auf einen Druck von 10 bis 16 bar abs. entspannt, wobei etwa 18 bis 70 Gew.-% des gesamten 1,2-Dichlorethans verdampfen;

c) das gemäß b) gewonnene dampfförmige 1,2-Dichlorethan vom flüssigen Anteil abtrennt, diesen flüssigen Anteil dann in einem außenliegenden Verdampfer bei einem Druck von 10 bis 16 bar abs. verdampft und die vereinigten Ströme an dampfförmigem Dichlorethan, in denen auch der Tetrachlorkohlenstoff in Dampfform zugegen ist, in solchen Mengen in die Reaktionszone des Spaltofens einführt, daß die stündliche Beaufschlagung pro m² Spaltrohrquerschnitt 1.100 bis 1.500 t 1,2-Dichlorethan beträgt;

d) 1,2-Dichlorethanumsätze von 60 bis 70 % bei einer mittleren Verweilzeit von 10 bis maximal 15 Sekunden, bezogen auf den befeuerten Teil der Reaktionszone, einstellt, indem man den Spaltofen so befeuert, daß im Bereich des Überganges von der Schock- in die Strahlungszone des Reaktionsraumes bereits ein Temperaturniveau von 425 bis 455 °C herrscht, etwa in der Mitte der Reaktionszone Temperaturen von 460 bis 480 °C herrschen und schon bei etwa 75 bis 85 % der gesamten Rohrlänge der Reaktionszone, bestehend aus Schock- und Strahlungsteil, der Rest der gesamten benötigten Energie zugeführt wird, um am Coilende Austrittstemperaturen von 485 bis 510 °C einzuhalten.

2. Verfahren nach Anspruch 1, **dadurch gekennzeichnet,** daß man den Wärmeinhalt der Rauchgase der Spaltofenbefeuerung, welche die Konvektionszone des Spaltofens mit einer Temperatur von 240 bis 540 °C verlassen, zur Überhitzung der etwa 100-grädigen Verbrennungsluft für die Spaltofenbefeuerung auf Temperaturen von 200 bis 500 °C unter gleichzeitigem Abkühlen der Rauchgase auf Temperaturen von 140 bis 180 °C ausnützt.

## Claims

1. Process for the production of vinyl chloride by thermal cracking of 1,2-dichloroethane, which is obtained in liquid form on purification by distillation at a temperature of 125°C to 155°C and corresponding pressures and, without intermediate storage and after the addition of carbon tetrachloride as cracking promoter, is vaporized in an external vaporizer, at temperatures of 400°C to 600°C under a pressure of 10 to 16 bar abs. and with conversions of 50 to 60% and residence times of 0.1 to 30 seconds in the reaction zone with partial utilization of the heat content of the flue gases from firing of the cracking furnace for pre-warming liquid 1,2-dichloroethane to virtually the boiling point under appropriate pressures before entry into the external vaporizer and using air pre-warmed to about 100°C for firing the cracking furnace, characterized in that

a) the carbon tetrachloride content in liquid 1,2-dichloroethane is kept at values between 0.10 and 0.15% by weight relative to 1,2-dichloroethane, and, at the same time, the chloroform content in liquid 1,2-dichloroethane is adjusted to values of less than 200 ppm by weight, relative to 1,2-dichloroethane;

b) the liquid 1,2-dichloroethane which is at 125 to 155°C and has been charged with carbon tetrachloride in accordance with a) is pre-warmed to virtually the boiling point under a liquid pressure of 15 to 31 bar abs. in the convention zone of the cracking furnace utilizing a large part of the heat content of the flue gases from firing the cracking furnace and, after issue from the convection zone of the cracking furnace, the pressure of the pre-warmed, liquid 1,2-dichloroethane is released to give a pressure of 10 to 16 bar abs., about 18 to 70% by weight of the total 1,2-dichloroethane vaporizing;

c) the 1,2-dichloroethane in vapour form obtained according to b) is separated from the liquid portion, this liquid portion is then vaporized in an external vaporizer under a pressure of 10 to 16 bar abs. and the combined streams of 1,2-dichloroethane in vapour form, in which the carbon tetrachloride is also present in vapour form, are fed into the reaction zone of the cracking furnace in amounts such that the hourly admission/m² cracking tube cross-section is 1100 to 1500 t 1,2-dichloroethane;

d) 1,2-dichloroethane conversions of 60 to 70% for an average residence time of 10 to at most 15 seconds, based on the fired portion of the reaction zone, are set by firing the cracking furnace such that a temperature level of 425 to 455°C already prevails in the transition region from the shock zone to the radiation zone of the reaction space, temperatures of 460 to 480°C prevail approximately in the middle of the reaction zone and the remainder of the total energy required is already supplied at about 75 to 85% of the total tube length of the reaction zone, consisting of shock section and radiation section, in order to maintain outlet temperatures 485 to 510°C at the coil end.

2. Process according to Claim 1, characterized in that the heat content of the flue gases from firing of the cracking furnace, which leave the convection zone of the cracking furnace at a temperature of 240 to 540°C, is used for super-heating the combustion air for firing the cracking furnace, which is at about 100°, to temperatures of 200 to 500°C, with simultaneous cooling of the flue gases to temperatures of 140 to 180°C.

## Revendications

1. Procédé pour préparer du chlorure de vinyle par craquage thermique du dichloro-1,2 éthane, que l'on obtient lors de la purification par distillation, à une température de 125°C à 155°C et sous des pressions correspondantes, sous forme liquide et que l'on évapore, sans magasinage intermédiaire ainsi qu'après addition de tétrachlorure de carbone comme promoteur de craquage, dans un évaporateur situé à l'extérieur, en opérant à des températures de 400 à 600°C sous une pression absolue de 10 à 16 bars ainsi qu'à des taux de réaction de 50 à 60% et à des temps de séjour de 0,1 à 30 s dans la zone de réaction, avec utilisation partielle de la chaleur contenue dans les gaz de fumée provenant du chauffage du four de craquage pour préchauffer le dichloro-1,2 éthane liquide jusqu'au voisinage de la température d'ébullition à la pression correspondante, avant l'entrée dans l'évaporateur externe et en utilisant de l'air préchauffé à environ 100°C, pour chauffer le four de craquage, procédé caractérisé en ce que

a) on maintient à des valeurs comprises entre 0,10 et 0,15% en poids, sur la base du dichloro-1,2 éthane, la teneur en du tétrachlorure de carbone dans le dichloro-1,2 éthane liquide, et, en même temps, on règle au-dessous de 200 mg, par kg de dichloro-1,2 éthane, la teneur en chloroforme dans le dichloro-1,2 éthane liquide;

b) on préchauffe jusqu'au voisinage de la température de l'ébullition, sous une pression absolue de liquide de 15 à 31 bars, le dichloro-1,2 éthane, liquide à 125 à 155° et qui a été additionné selon a) de tétrachlorure de carbone, dans la zone de convection du four de craquage, en utilisant une grande partie de la chaleur contenue dans les gaz fumée provenant du chauffage de ce four de craquage et, après sa sortie de la zone de convection du four de craquage, on détend, jusqu'à une pression absolue de 10 à 16 bars, le dichloro-1,2 éthane liquide préchauffé, ce qui provoque l'évaporation d'environ 18 à 70% en poids de la totalité du dichloro-1,2 éthane;

c) on sépare le dichloro-1,2 éthane obtenu selon b) sous forme de vapeur, de la partie liquide, on évapore ensuite cette partie liquide, sous une pression absolue de 10 à 16 bars, dans un évaporateur situé à l'extérieur et l'on introduit les courants réunis du dichloroéthane sous forme de vapeur, dans lesquels on a également ajouté le tétrachlorure de carbone sous forme de vapeur, en des quantités telles dans la zone de réaction du four de craquage que l'alimentation horaire, par m³ de section de tube de craquage, soit de 1100 à 1500 t de dichloro-1,2 éthane;

d) on ajuste entre 60 et 70% le taux de réaction du dichloro-1,2 éthane, pour un temps moyen de séjour de 10 s à 15 s au maximum, par rapport à la partie chauffée de la zone de réaction, en chauffant le four de craquage de manière que, dans la région de passage de la zone de choc à la zone de rayonnement de l'espace de réaction, il règne déjà un niveau de température de 425 à 455°C, environ au milieu de la zone de réaction il règne des températures de 460 à 480°C et déjà à environ 75 à 85% de la longueur totale du tube de la zone de réaction, consistant en la partie choc et la partie de rayonnement, on achemine le reste de l'énergie totale nécessaire pour maintenir à l'extrémité du ou des serpentins des températures de sortie de 485 à 510°C.

2. Procédé selon la revendication 1, caractérisé en ce qu'on utilise la chaleur contenue dans les gaz de fumée provenant du chauffage du four de craquage, qui quittent la zone de convection du four de craquage en ayant une température de 240 à 540°C, pour surchauffer, jusqu'à des températures de 200 à 500°C, l'air comburant, qui se trouve à environ 100°, pour le chauffage du four de craquage, en refroidissant simultanément les gaz de fumée jusqu'à des températures de 140 à 180°C.

Figur 1

EP 0 225 617 B1